Europäisches Patentamt

European Patent Office

Office européen des brevets

(11) Publication number: 0 375 011 A1

## EUROPEAN PATENT APPLICATION

(21) Application number: 89203099.0

(51) Int. Cl.5: **A61N 5/06, F21S 1/12**

(22) Date of filing: 06.12.89

(30) Priority: **07.12.88 NL 8803005**

(43) Date of publication of application:
**27.06.90 Bulletin 90/26**

(84) Designated Contracting States:
**AT BE CH DE ES FR GB IT LI LU NL SE**

(71) Applicant: **DESTIM PRODUCTS B.V.**
**Gironde 12**
**NL-3831 AB Leusden(NL)**

(72) Inventor: **Emmelkamp, Folkert Johannes**
**Flevo 134**
**NL-9204 JS Drachten(NL)**

(74) Representative: **Smulders, Theodorus A.H.J.,**
**Ir. et al**
**Vereenigde Octrooibureaux Nieuwe Parklaan**
**107**
**NL-2587 BP 's-Gravenhage(NL)**

(54) Tanning apparatus.

(57) The invention relates to a tanning apparatus comprising a table part (1) and a hand part (7). The table part (1) comprises a ballast circuit for exciting one or more UV-lamps and a resting-place (3) for the hand part (7). The hand part (7) comprises a lamp holder containing one or more UV-lamps and is linked to the table part by means of a connecting cord (6). Preferably, the lamps are sunk in the hand part (1) and the resting-place is a recess (3) in the table part (1). The recess (3) in turn may have a sunk bottom portion optionally containing a partition for accommodating the connecting cord (6) and preferably providing a means for supporting the hand part in an upright service position.

FIG.10

## Tanning apparatus

The invention relates to a tanning apparatus for locally tanning the skin with the help of one or more lamps emitting when in operation ultra-violet radiation, comprising a lamp holder and a ballast circuit for exciting the lamp(s).

Tanning apparatuses can be employed for cosmetic and/or medical purposes, such as the treatment of psoriasis, and are generally known in the form of sunbeds and sun canopies.

Sunbeds and sun canopies comprise a number of relatively long tubular fluorescent lamps and are designed to irradiate the full length of the human body at the same time. As a result sunbeds and sun canopies occupy a great deal of space and therefore installing them will in most houses meet with serious objections. Moreover, sunbeds and sun canopies are not suitable for irradiating only a part of the body, for instance the face or the arms. The traditional sun lamp has this same drawback.

There are bar-shaped tanning apparatuses available on the market, which have to be held in the hand and are suitable for treating only a part of the body. These known hand apparatuses have an essentially tubular body made of plastic, comprising a part that serves as a grip, and contains a ballast circuit, and a part that is co-extensive with the part serving as a grip, and contains a suitable compact fluorescent lamp. At the position of the lamp, the tubular body is provided with an opening or window which, in operation, is held before the part of the body to be treated, so that the ultraviolet radiation may impinge upon the skin.

A drawback of the known bar-shaped tanning apparatus is that in operation it has to be held in the hand continuously, and that due to the presence of a ballast circuit in the grip part it is comparatively heavy. Furthermore, the lamp in a bar-shaped apparatus is rather vulnerable so the apparatus has to be fitted with a transparent cover to protect the lamp. As a result, however, the apparatus will be less efficient.

The present invention aims to overcome the abovementioned drawbacks and, generally to provide a tanning apparatus simple and efficient in use. To this effect, a tanning apparatus of the kind described, is characterized, according to the present invention, in that the ballast circuit is accommodated in a table part of the tanning apparatus and the lamp holder constitutes a hand part of the tanning apparatus, which hand part is linked to the table part by means of an electrical connecting cord, the table part being provided with a resting place for accommodating the hand part when at rest.

One embodiment of the invention will now be described, by way of example, with reference to the accompanying drawings, in which:

Fig. 1 shows schematically in top view an example of a part of a tanning apparatus according to the present invention;

Fig. 2 schematically shows a part-sectional elevation as indicated by the arrow II in Fig. 1;

Figs. 3 and 4 schematically show, in front-elevational view and side-elevational view, respectively, an example of a hand part of a tanning apparatus according to the invention;

Fig. 5 shows a cross-section taken on the line V-V of Fig. 3;

Figs. 6-8 are top, side, and front views, illustrating an example of a complete tanning apparatus according to the invention in the inoperative position;

Fig. 9 schematically shows a cross-section taken on the line IX-IX of Fig. 7; and

Fig. 10 shows a possible service position of a tanning apparatus according to the invention.

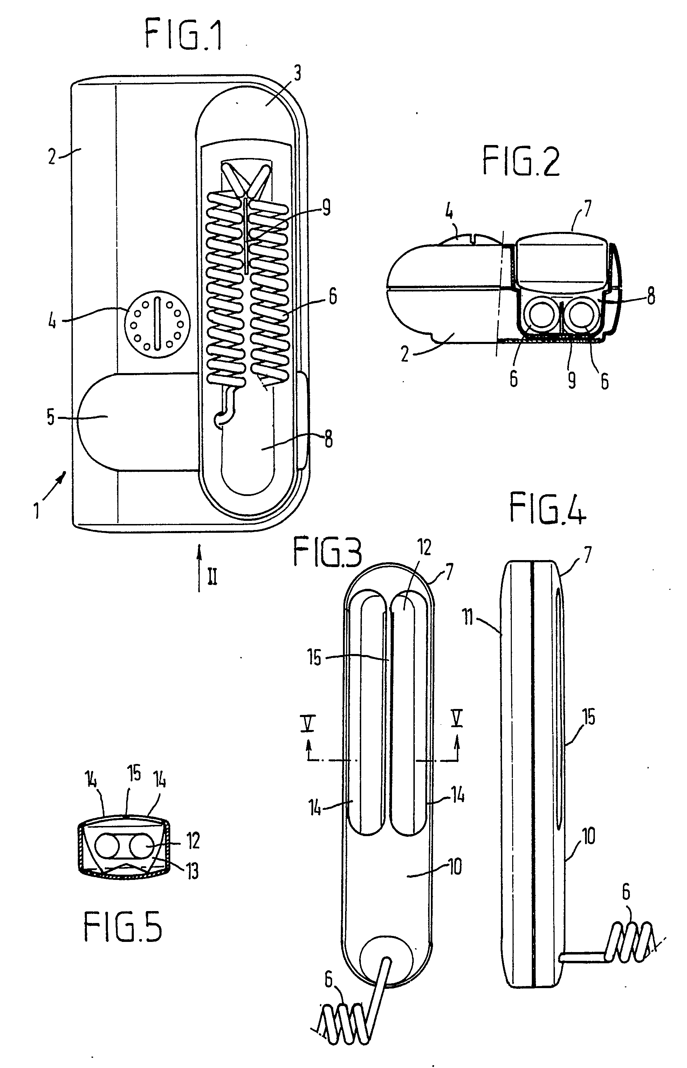

Fig. 1 shows schematically in top plan view a table part 1 of a tanning apparatus according to the invention. The table part comprises a box-shaped body 2 - in this example in top view essentially rectangular in shape - provided with a recess 3, which constitutes a resting-place for a hand part of the tanning apparatus.

The body contains a ballast circuit known per se for exciting the lamp(s) of the tanning apparatus (not shown in the drawing). The tanning apparatus is further provided with an electrical cord (not shown) or alternatively, a socket for a plug. In the example shown, a control knob 4 for an on/off-switch is provided on the top of the body.

Further, in the example shown, the body 2 is provided on top with a recess 5 extending on both sides of the resting place 3, to facilitate gripping the seated hand part. Such a recess is particularly useful if the hand part at rest is for the greater part sunk in the table part as shown in the example. As an alternative to, or in combination with, a recess 5 in the table part, the hand part may be provided with recesses on both sides.

Fig. 1 also shows a connecting cord 6 which links the hand part to the table part. The connecting cord may advantageously be passes through an opening made in a wall of resting-place 3 and the resting-place may be designed in such a way that the connecting cord can be accommodated in the resting-place along with the hand part.

Fig. 2 shows in what way a hand part 7 of a tanning apparatus according to the invention can be accommodated in the resting-place of the table part. Fig. 2 shows furthermore in what way the connecting cord in this example is accommodated in a sunk portion of the resting-place under hand part 7 in the inoperative position.

To orderly accommodate the cord in the resting-place, in this example the sunk portion of the resting-place is provided with a partition 9, which extends vertically from the bottom of the sunk portion of the resting-place and lengthwise in the resting-place. At one end at least the partition 9 does not extend to the corresponding end of the sunk portion, so that the cord can be laid around the partition, as can be seen in Fig. 1.

The hand part is shown in more detail in Figs. 3, 4 and 5. The hand part is elongate and more or less rectangular in shape, in this example with the corners rounded off and a somewhat convex top wall 10 and bottom wall 11. The hand part is made of a suitable plastic and, as mentioned before, is linked to the table part by means of a connecting cord 6. The connecting cord 6 in this example is a spiral cord. In the inoperative position, top wall 10 of the hand part faces the sunk portion of the resting-place and thus constitutes the bottom of the hand part.

The hand part comprises at least one suitable lamp 12, for instance a compact fluorescent lamp of type CF-S 9W/BL 350 UV-A, and a suitable reflector 13. At the position of the lamp, the hand part is provided with an opening, which, in the embodiment shown, comprises two elongate openings 14 extending in side-by-side relationship in the longitudinal direction of the hand part and separated by a rib 15.

The lamp is somewhat set back from openings 14 and thus cannot come into contact with the skin. This effect is enhanced still further by the provision of rib 15.

This design also effectively protects the lamp against being damaged when the hand part is put on the table or placed in the table part. For this reason there is no need for an additional protective cover, either when the tanning apparatus is being used, or when it is at rest.

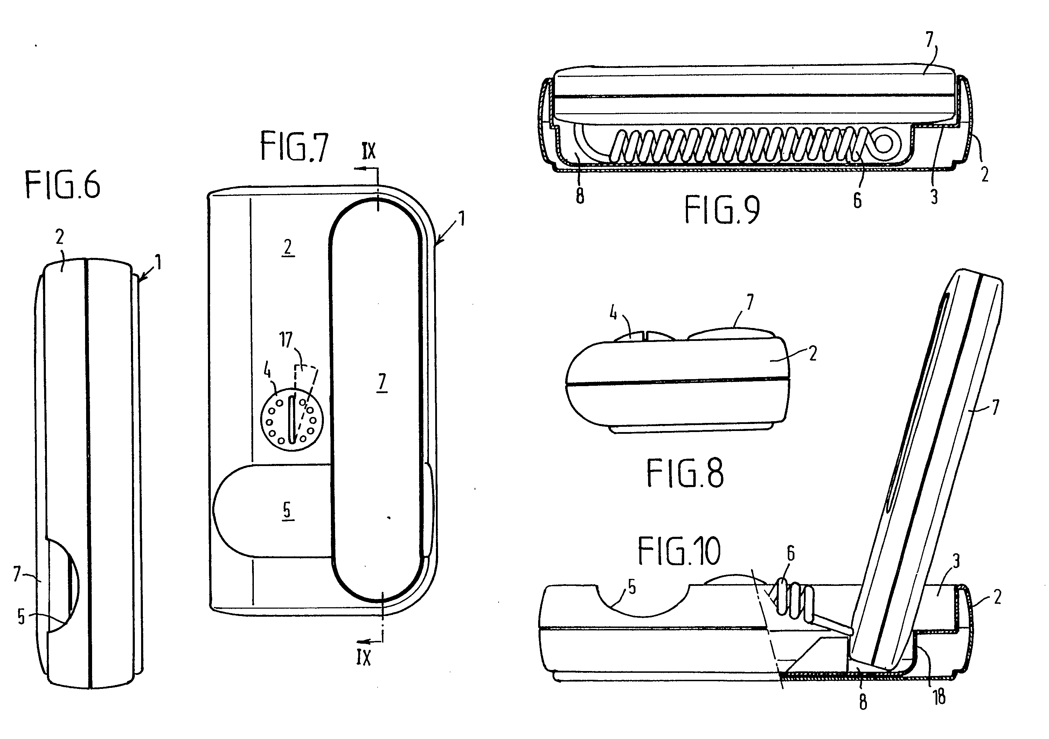

Figs. 6, 7 and 8 show a tanning apparatus according to the invention in the inoperative position, i.e. the condition where the hand part 7 is placed in the resting-place of the table part. It is clearly shown that in this embodiment the hand part when at rest is for the greater part sunk in the table part. In this way a compact apparatus is obtained, of which the hand part, and in particular the lamp(s) when at rest are well-protected.

If so desired, any additional electronic circuits can readily be added to the table part of the tanning apparatus according to the invention. The table part could advantageously be fitted with a timer enabling the period of time during which the apparatus is to operate to be set prior to each treatment. Such a timer may at the same time function as an on/off switch and the control knob 4 could then be a rotary switch, which, as shown, is provided with a scale or the like.

The control knob 4 may further be connected to a mechanical catch, which extends into resting-place 3 when the apparatus is in operation and thus prevents the hand part from being placed in the resting-place as long as the lamps are energized.

The broken lines 17 in Fig. 7 show an example of such a safety catch, which is fitted onto the shaft of the rotary switch within the body 2 and thus rotates along with the rotary switch. Such safety catches or similar safety means are particularly useful in apparatuses without timers.

Fig. 9 is a cross-sectional view taken on the line IX-IX of Fig. 7, showing, a tanning apparatus according to the invention when it is at rest, in which the hand part 7 is accommodated in the sunk portion 8 of the resting-place 3 together with the connecting cord 6.

Fig. 10 shows schematically in side view an embodiment of a tanning apparatus according to the invention, where the table part is provided with means for supporting the hand part in a stable manner and in a suitable service position.

In the embodiment shown, the sunk portion of the resting-place is of such width, at least at one end of the sunk portion, that the bottom end of the hand part, i.e. the closed end thereof, can neatly be fitted into the sunk portion while leaning against the upright end edge 18 of the sunk portion. The hand part is prevented from tipping backwards by a support member provided at a suitable distance from the upright end edge 18, which support member could advantageously be constituted by the partition 9 described above.

It is noted that after reading the above various modifications will readily occur to those skilled in the art. Thus the form of both the table part and the hand part may be modified without departing from the scope of the invention. Also, the body 2 could for instance be fitted with a pilot light, or the switch and/or timer know could be provided at a different place, etc.

Furthermore, to orderly accommodate the connecting cord and/or to support the hand part in a service position, other means could be used instead of the partition 9, for instance one or more bar-shaped projections. These and similar modifications are considered as falling within the scope of the present invention.

## Claims

1. A tanning apparatus for locally tanning the

3

skin with the help of one or more lamps emitting when in operation ultra violet radiation, comprising a lamp holder and a ballast circuit for exciting the lamp(s), characterized in that the ballast circuit is fitted in a table part of the tanning apparatus and that the lamp holder constitutes a hand part of the tanning apparatus, which hand part is linked to the table part by means of an electrical connecting cord, the table part being provided with a resting place for accommodating the hand part when at rest.

2. A tanning apparatus according to claim 1, characterized in that the resting-place is a recess in the table part, which recess has a shape which is essentially complementary to the outward shape of the hand part, the hand part when at rest being for the greater part sunk in the recess.

3. A tanning apparatus according to claim 2, characterized in that the table part is provided with a sunk recess extending on both sides of the resting-place to facilitate gripping the hand part.

4. A tanning apparatus according to claim 2 or 3, characterized in that the hand part is provided on both sides with recesses to facilitate gripping the hand part.

5. A tanning apparatus according to any one of the preceding claims, characterized in that the resting-place is provided with a sunk bottom portion for accommodating the electric connecting cord.

6. A tanning apparatus according to claim 5, characterized in that the sunk bottom portion is fitted with ordering means for ordering the connecting cord.

7. A tanning apparatus according to claim 6, characterized in that the ordering means comprises a partition rising from the bottom of the sunk bottom portion, there being an empty space between at least one end of the partition and the correspond end edge of said sunk portion.

8. A tanning apparatus according to any one of the preceding claims, characterized in that the table part is provided with means for supporting the hand part in a substantially upright service position.

9. A tanning apparatus according to claim 8 and 5, characterized in that the sunk bottom portion is shaped at least in part as a holding part of such width that an end of the hand part can be placed in said holding part for supporting the hand part in an upright service position.

10. A tanning apparatus according to claim 9, characterized in that the holding part is formed by an end part of the sunk bottom portion.

11. A tanning apparatus according to claim 10, characterized in that the holding part is bounded by the edge of the end part of the sunk portion and by at least one supporting means fitted in said sunk portion.

12. A tanning apparatus according to claim 11 and 6 or 7, characterized in that the supporting means is at least part of the ordering means.

13. A tanning apparatus according to any one of the preceding claims, characterized in that the hand part is an essentially closed elongate hollow body, containing at least one lamp holder with a lamp, there being provided at least one hole in the wall of the body, the lamp being set back behind said at least one hole.

14. A tanning apparatus according to claim 13, characterized in that the hand part is provided with two elongate and essentially parallel holes, which are separated by a rib.

15. A tanning apparatus according to any one of the preceding claims, characterized in that the table part is provided with safety means for preventing the hand part from being placed at rest in the resting-place with the lamp still switched on.

16. A tanning apparatus according to any one of the preceding claims, characterized in that the safety means comprise a catch, which is connected to a control knob of an on/off-switch fitted onto the table part, and extends into the resting-place when the switch is in the "on"-position.

17. A tanning apparatus according to claim 16, characterized in that the catch is fitted onto the shaft of the on/off-switch, which is a rotary switch.

18. A tanning apparatus according to any one of the preceding claims, characterized in that the table part is fitted with an adjustable timer.

FIG.1

FIG.2

FIG.3

FIG.4

FIG.5

FIG.6

FIG.7

FIG.8

FIG.9

FIG.10

EP 0 375 011 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | NL-A-6815450 (ORIGINAL HANAU QUARZLAMPEN)<br>* page 4, line 4 - page 6, line 33; figures * | 1-3,<br>8-11,<br>15-17 | A61N5/06<br>F21S1/12 |
| A | DE-A-3140258 (WERNER SCHOTT ELEKTROGERÄTE)<br>* page 7, line 1 - page 9, line 16; figures * | 1, 2, 8,<br>9-11, 13 | |
| A | US-A-3265882 (NAXON)<br>* the whole document * | 1, 2,<br>5-8 | |
| A | CH-A-410212 (KERN & SPRENGER)<br>* page 1, line 65 - page 2, line 14; figures * | 1, 8, 18 | |

| | |
|---|---|
| | TECHNICAL FIELDS SEARCHED (Int. Cl.5 ) |
| | A61N<br>F21S |

The present search report has been drawn up for all claims

| Place of search | Date of completion of the search | Examiner |
|---|---|---|
| THE HAGUE | 16 MARCH 1990 | LEMERCIER D.L.L. |